# EUROPEAN PATENT APPLICATION

(11) **EP 4 406 934 A1**
(43) Date of publication of application: **31.07.2024**
(21) Application number: 22870281.7
(22) Date of filing: 14.09.2022
(51) Int. Cl.: C07C 39/23, C07C 39/373, C07C 323/16, C07C 323/18, C07C 245/08, C07C 255/33, C07C 205/16, C07C 311/29, C07C 329/06, C07C 43/23

(54) **NOVEL CANNABIDIOL DERIVATIVE, METHOD FOR PREPARING SAME, AND COMPOSITION FOR COGNITIVE FUNCTION IMPROVEMENT COMPRISING SAME**

(30) Priority: 15.09.2021 KR 20210123378
(71) Applicant: Kangwon University-Industry Cooperation Foundation, Chuncheon-si, Gangwon-do 24341 (KR)
(72) Inventor: LEE, Koo Yeon, Chuncheon-si, Gangwon-do 24341 (KR)
(74) Representative: Graf von Stosch Patentanwaltsgesellschaft mbH
(86) International application number: PCT/KR2022/013736
(87) International publication number: WO 2023/043197

(57) **Abstract**

The present invention provides a cannabidiol derivative, a method for preparing same, and a composition for cognitive function improvement comprising same. The novel cannabidiol derivative of the present invention has been confirmed to exhibit acetylcholinesterase inhibitory activity and butyrylcholinesterase inhibitory activity, as well as inhibitory activity against monoamine oxidase B. Therefore, it is possible to simultaneously treat a decrease in acetylcholine levels in brain tissue and a decrease in blood butyrylcholine levels, which are seen in patients with dementia, and to provide a more effective cognitive function improvement treatment by preventing brain cell damage.

Therefore, the cannabidiol derivative, the method for preparing same, and the composition for improving cognitive function comprising same, of the present invention, can be usefully used as a substance for improving cognitive function in the field of medicine and dietary supplement that can simultaneously inhibit acetylcholinesterase, butyrylcholinesterase, and monoamine oxidase B.

## Description

### FIELD

The present invention relates to novel cannabidiol derivative, and more specifically, to novel cannabidiol derivative, method for preparing same, and composition for cognitive function improvement comprising same.

### BACKGROUND

As we enter an aging society, the number of people living with dementia is increasing and the health and social costs are rising. As a result, dementia is increasingly becoming a national issue rather than an individual problem. Dementia is a pathological phenomenon that must be distinguished from normal aging, and depending on the cause, it is divided into Alzheimer's disease, vascular dementia, other dementias such as alcoholism, trauma, and sequelae of Parkinson's disease, and more than 60% of dementia cases are known to be Alzheimer's disease. Dementia is characterized by a decline in intellectual functions such as memory, judgment, and language, a decline in daily living skills, and clinical features such as personality or behavioral disorders.

The pathogenesis of Alzheimer's disease is not clearly understood, but the toxicity of neurotoxic protein β-amyloid has been proposed as the most important cause. It is believed to be produced by improper metabolism of amyloid precursor protein (APP), while normal metabolites of APP have been reported to be neuroprotective. Recent studies using genetic engineering methods have shown that toxic proteins such as beta-amyloid (β-amyloid) accumulate in cells and blood vessels and are toxic to neurons, resulting in impaired brain function.

To date, pharmacologic treatments for dementia have been based on increasing acetylcholine concentrations in the brain by administering acetylcholine precursors or drugs that inhibit the breakdown of acetylcholine. Typical drugs include tacrine, donepezil, rivastigmine, and galantamine.

These drugs are acetylcholinesterase inhibitor-based compounds that are currently used to treat dementia and are known to have side effects in the digestive system, including nausea, vomiting, loss of appetite, and abdominal pain. In addition, as the activity of butyrylcholinesterase is dramatically increased in Alzheimer's disease, there is an urgent need to develop simultaneous inhibitors that not only inhibit acetylcholinesterase activity but also butyrylcholinesterase activity in order to treat Alzheimer's disease more effectively.

Meanwhile, monoamine oxidase (MAO) is an enzyme that catalyzes the oxidative deamination of neurotransmitters such as dopamine, serotonin, adrenaline, and noradrenaline, as well as non-biogenic amines, and there are two types of MAO-A and MAO-B. Neurodegenerative diseases such as Alzheimer's and Parkinson's are caused by oxidative stress caused by hydrogen peroxide from increased MAO activity, and MAO-B inhibitors can reduce oxygen radical formation and increase the amount of useful monoamines in the brain. Furthermore, in brain diseases and brain injury, including Alzheimer's disease, MAO-B promotes putrescine metabolism within reactive astrocytes, leading to the production of excess GABA. Therefore, MAO-B inhibitors may act as inhibitors of GABA production by astrocytes to restore neural signaling and brain function, which may help improve symptoms of brain diseases and brain injury.

### [Prior art references]

### [Patent Literature]

US Patent US 11207292 B2 (Dec. 28, 2021)

### [Non-Patent Literature]

Georgia Watt., Tim Karl. In vivo Evidence for Therapeutic Properties of Cannabidiol (CBD) for Alzheimer's Disease, Frontiers in Pharmacology, v.8, Article 20, (2017)

### OBJECT OF THE INVENTION

The problem to be addressed by the present invention is to provide a compound that simultaneously inhibits acetylcholinesterase activity and butyrylcholinesterase activity to more effectively improve cognitive dysfunction such as Alzheimer's dementia.

Further, the problem to be addressed by the present invention is to provide a compound that improves cognitive dysfunction such as degenerative brain disease by inhibiting monoamine oxidase (MAO).

Furthermore, the problem to be addressed by the present invention is to provide a pharmaceutical composition or food composition comprising the compound showing the cognitive function improvement effect, which can be used for the purpose of improving cognitive function.

Furthermore, the problem to be addressed by the present invention is to provide a preparing method that can more efficiently synthesize the compound showing the cognitive function improvement effect.

The problem to be addressed by the present invention is not limited to the problems mentioned above, and other technical problems not mentioned can be clearly understood by those skilled in the art from the description below. There will be.

### SUMMARY

To address the problem above, according to one aspect of the present invention, there is provided a cannabidiol derivative compound represented by the following Formula 1, a hydrate thereof, a solvate thereof or a pharmaceutically acceptable salt thereof, wherein: 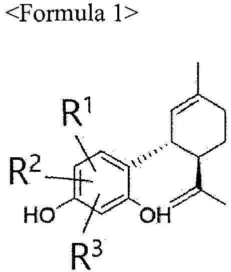
R¹, R², R³ are independently hydrogen, C₁-C₆ straight or branched chain alkyl, C₁-C₄ alkoxy, halogen, cyano, hydroxy, carboxyl, acetyl, dipentenyl, CONHR^{2a} or arylalkyl,
R^{2a} is arylalkyl substituted with hydroxy or alkoxy.

According to another aspect of the present invention, there is provided a pharmaceutical composition for cognitive function improvement, comprising the cannabidiol derivative compound represented by the above Formula 1, a hydrate thereof, a solvate thereof or a pharmaceutically acceptable salt thereof as an active ingredient.

According to another aspect of the present invention, there is provided a pharmaceutical composition for cognitive function improvement, characterized in that the cognitive function improvement is preventing or treating degenerative brain disease.

According to another aspect of the present invention, there is provided a composition for inhibiting monoamine oxidase (MAO) comprising the cannabidiol derivative compound represented by the above Formula 1, a hydrate thereof, a solvate thereof or a pharmaceutically acceptable salt thereof as an active ingredient.

According to another aspect of the present invention, there is provided a food composition for cognitive function improvement comprising the cannabidiol derivative compound represented by the above Formula 1, a hydrate thereof, a solvate thereof or a pharmaceutically acceptable salt thereof as an active ingredient.

According to another aspect of the present invention, there is provided a method for preparing a cannabidiol derivative compound represented by the following Formula 1 , comprising: obtaining a first mixture by mixing a benzoic acid compound represented by Formula 2 below and cis-isolimonenol represented by Formula 3 below; reacting the obtained first mixture with p-toluenesulfinic acid or oxalic acid hydrate to obtain a second mixture comprising a compound represented by Formula 4 below and a compound represented by Formula 1 below; and purifying the obtained second mixture to obtain a cannabidiol derivative compound represented by Formula 1 below. 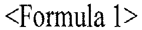 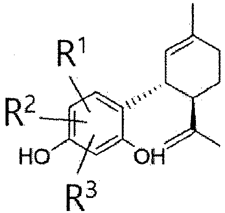 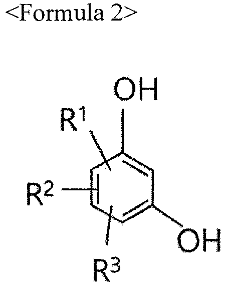 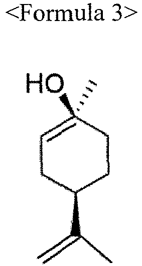 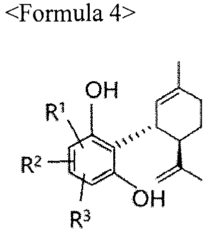 wherein in Formula 1, Formula 2, Formula 3 and Formula 4, R¹, R² and R³ are the same as defined above.

The novel cannabidiol derivatives of the present invention have been confirmed to exhibit acetylcholinesterase inhibitory and butyrylcholinesterase inhibitory effects, as well as inhibitory effects on monoamine oxidase B. It has been found that it is possible to simultaneously treat a decrease in the level of acetylcholine in the brain tissue and a decrease in the level of butyrylcholine in the blood, which are seen in patients with dementia, and to treat degenerative brain diseases more effectively by preventing brain cell damage, and that the amount of each drug can be reduced, thereby reducing side effects in the treatment of dementia.

Therefore, the cannabidiol derivative of the present invention, a method of preparing the same, and a composition for improving cognitive function comprising the same as an active ingredient can be usefully used as a substance for improving cognitive function in the field of medicine and dietary supplement that can simultaneously inhibit acetylcholinesterase, butyrylcholinesterase and monoamine oxidase B.

The effects of the present invention are not limited to the above effects, but should be understood to include all effects that can be inferred from the description of the invention or the composition of the invention as recited in the patent claims.

### BRIEF DESCRIPTION OF THE DRAWINGS

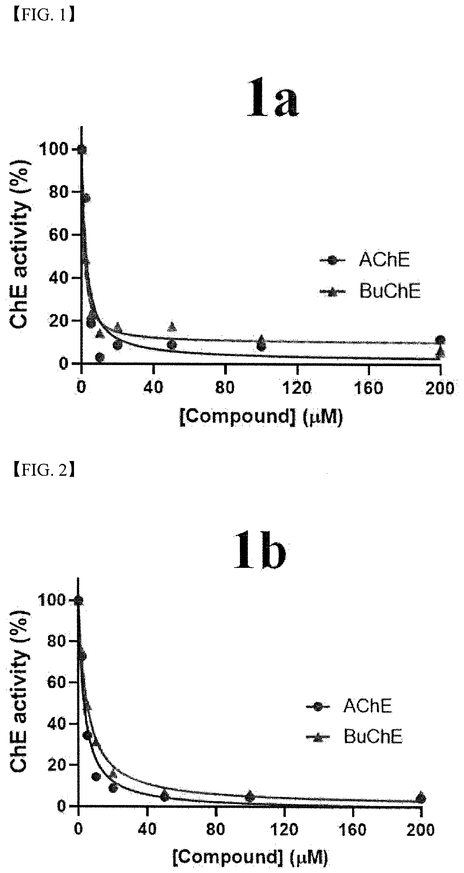

FIG. 1 is a graph showing the acetylcholinesterase (AChE) inhibition efficiency (IC₅₀) and butyrylcholinesterase (BuChE) inhibition efficiency (IC₅₀) of the synthesized cannabidiol derivative (1a).
FIG. 2 is a graph showing the acetylcholinesterase (AChE) inhibition efficiency (IC₅₀) and butyrylcholinesterase (BuChE) inhibition efficiency (IC₅₀) of the synthesized cannabidiol derivative (1b).

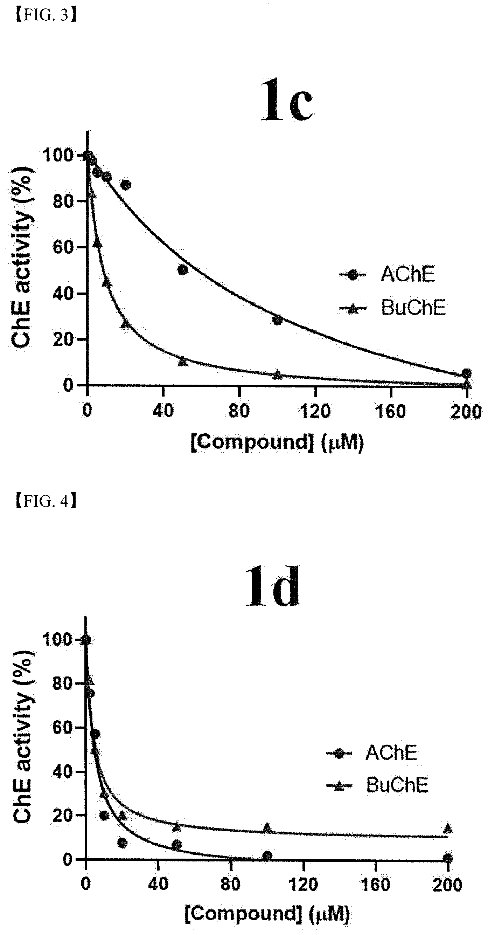

FIG. 3 is a graph showing the acetylcholinesterase (AChE) inhibition efficiency (IC₅₀) and butyrylcholinesterase (BuChE) inhibition efficiency (IC₅₀) of the synthesized cannabidiol derivative (1c).
FIG. 4 is a graph showing the acetylcholinesterase (AChE) inhibition efficiency (IC₅₀) and butyrylcholinesterase (BuChE) inhibition efficiency (IC₅₀) of the synthesized cannabidiol derivative (1d).

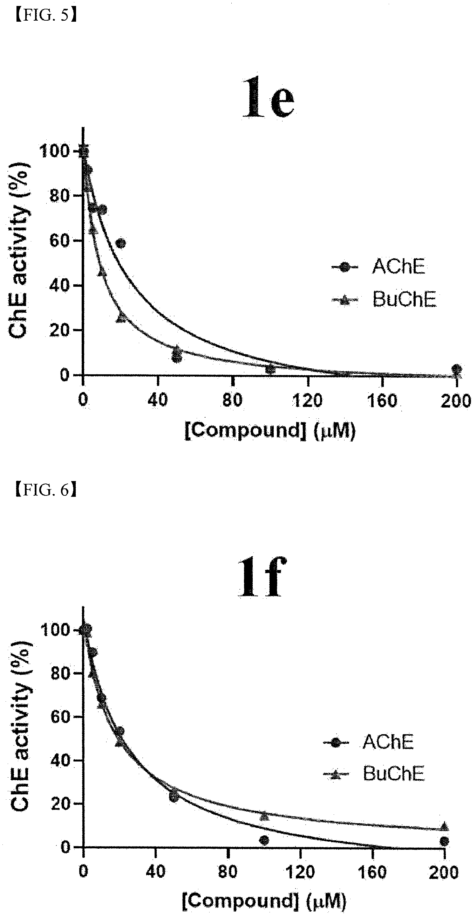

FIG. 5 is a graph showing the acetylcholinesterase (AChE) inhibition efficiency (IC₅₀) and butyrylcholinesterase (BuChE) inhibition efficiency (IC₅₀) of the synthesized cannabidiol derivative (1e).
FIG. 6 is a graph showing the acetylcholinesterase (AChE) inhibition efficiency (IC₅₀) and butyrylcholinesterase (BuChE) inhibition efficiency (IC₅₀) of the synthesized cannabidiol derivative (1f).

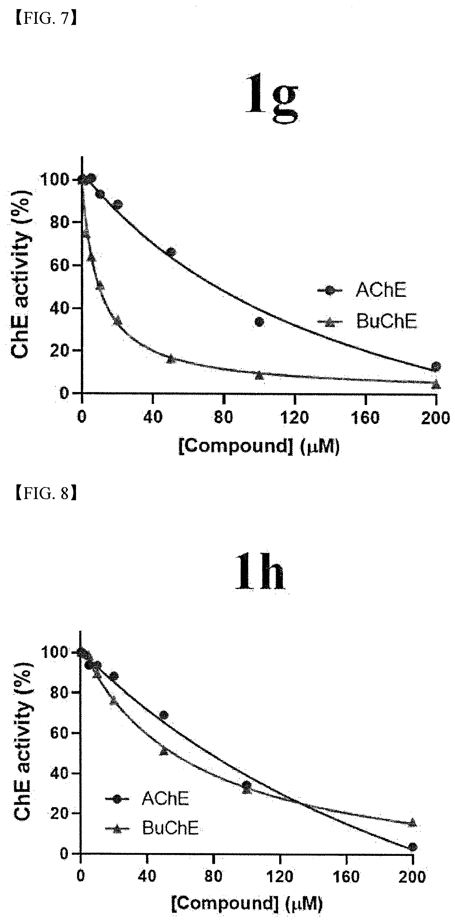

FIG. 7 is a graph showing the acetylcholinesterase (AChE) inhibition efficiency (IC₅₀) and butyrylcholinesterase (BuChE) inhibition efficiency (IC₅₀) of the synthesized cannabidiol derivative (1g).
FIG. 8 is a graph showing the acetylcholinesterase (AChE) inhibition efficiency (IC₅₀) and butyrylcholinesterase (BuChE) inhibition efficiency (IC₅₀) of the synthesized cannabidiol derivative (1h).

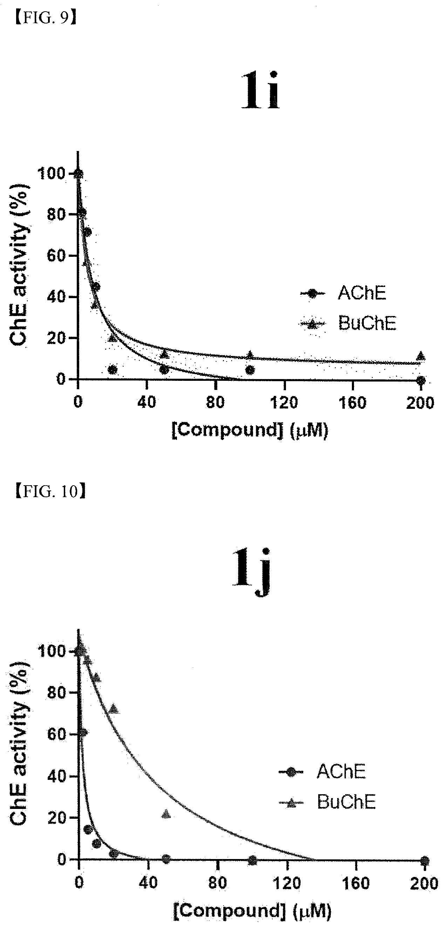

FIG. 9 is a graph showing the acetylcholinesterase (AChE) inhibition efficiency (IC₅₀) and butyrylcholinesterase (BuChE) inhibition efficiency (IC₅₀) of the synthesized cannabidiol derivative (1i).
FIG. 10 is a graph showing the acetylcholinesterase (AChE) inhibition efficiency (IC₅₀) and butyrylcholinesterase (BuChE) inhibition efficiency (IC₅₀) of the synthesized cannabidiol derivative (1j).

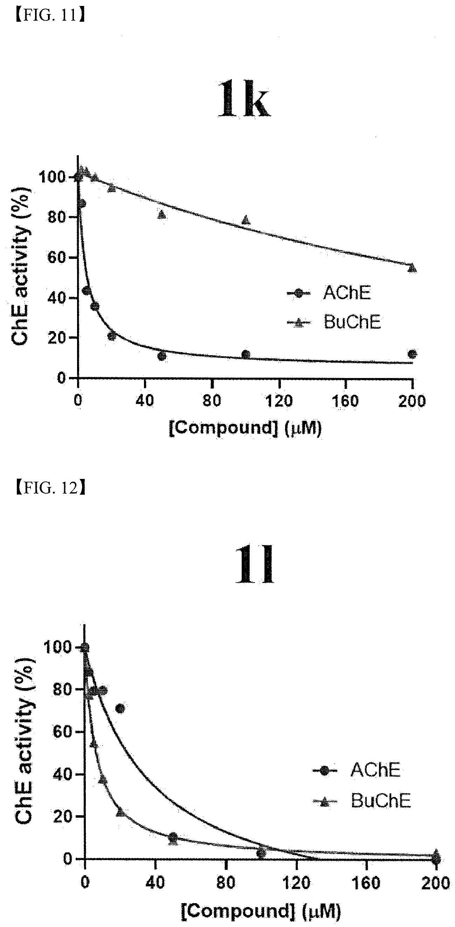

FIG. 11 is a graph showing the acetylcholinesterase (AChE) inhibition efficiency (IC₅₀) and butyrylcholinesterase (BuChE) inhibition efficiency (IC₅₀) of the synthesized cannabidiol derivative (1k).
FIG. 12 is a graph showing the acetylcholinesterase (AChE) inhibition efficiency (IC₅₀) and butyrylcholinesterase (BuChE) inhibition efficiency (IC₅₀) of the synthesized cannabidiol derivative (11).

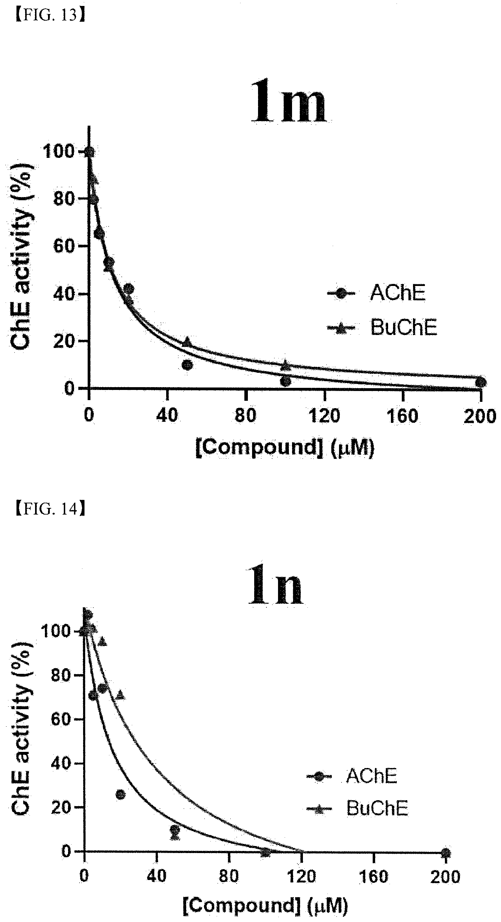

FIG. 13 is a graph showing the acetylcholinesterase (AChE) inhibition efficiency (IC₅₀) and butyrylcholinesterase (BuChE) inhibition efficiency (IC₅₀) of the synthesized cannabidiol derivative (1m).
FIG. 14 is a graph showing the acetylcholinesterase (AChE) inhibition efficiency (IC₅₀) and butyrylcholinesterase (BuChE) inhibition efficiency (IC₅₀) of the synthesized cannabidiol derivative (1n).

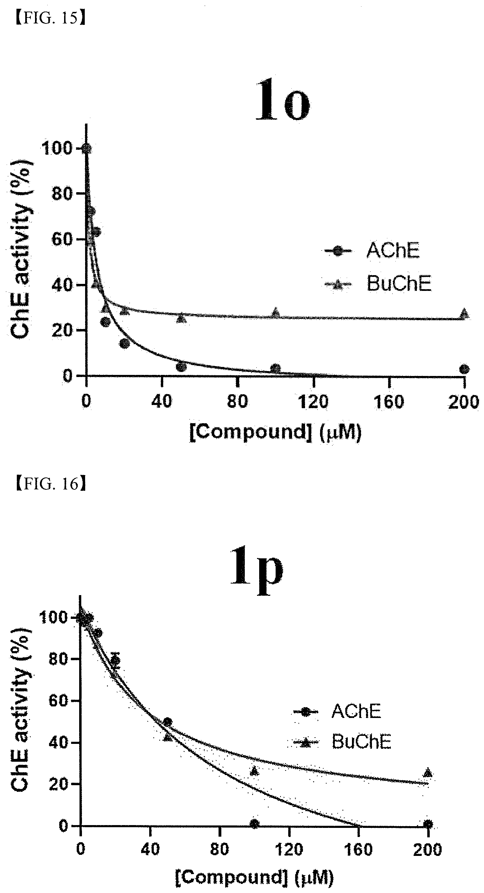

FIG. 15 is a graph showing the acetylcholinesterase (AChE) inhibition efficiency (IC₅₀) and butyrylcholinesterase (BuChE) inhibition efficiency (IC₅₀) of the synthesized cannabidiol derivative (1o).
FIG. 16 is a graph showing the acetylcholinesterase (AChE) inhibition efficiency (IC₅₀) and butyrylcholinesterase (BuChE) inhibition efficiency (IC₅₀) of the synthesized cannabidiol derivative (1p).

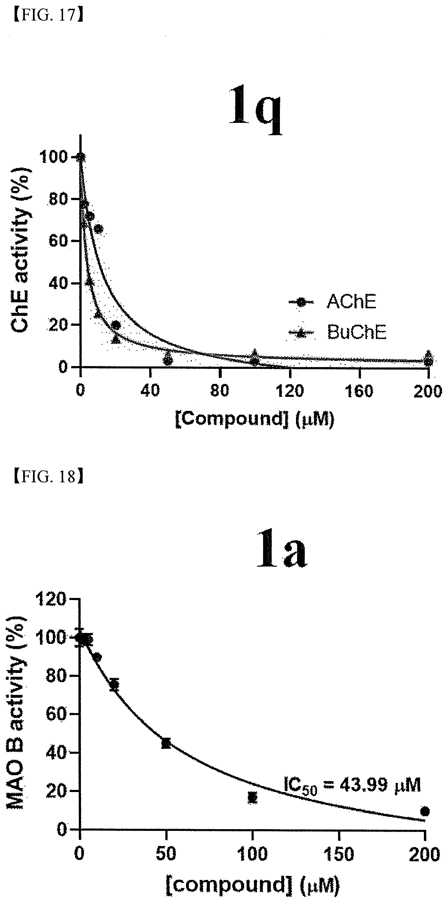

FIG. 17 is a graph showing the acetylcholinesterase (AChE) inhibition efficiency (IC₅₀) and butyrylcholinesterase (BuChE) inhibition efficiency (IC₅₀) of the synthesized cannabidiol derivative (1q).
FIG. 18 is a graph showing the monoamine oxidase (MAO) inhibition efficiency (IC₅₀) of the synthesized cannabidiol derivative (1a).

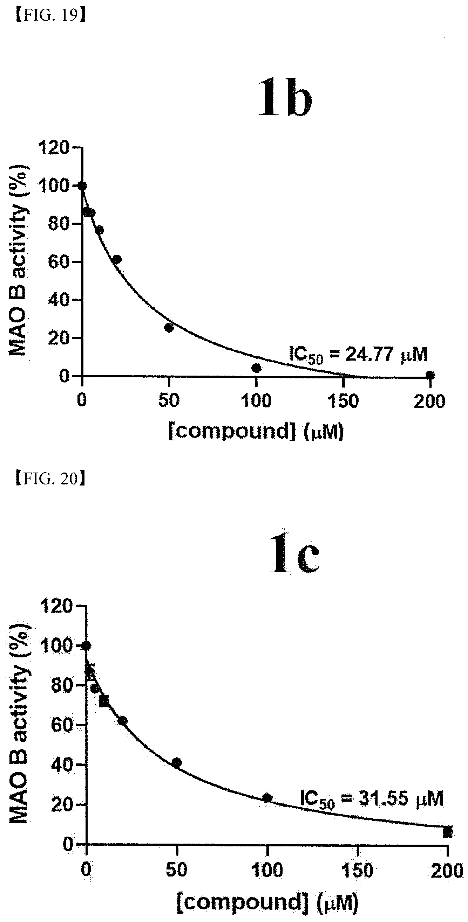

FIG. 19 is a graph showing the monoamine oxidase (MAO) inhibition efficiency (IC₅₀) of the synthesized cannabidiol derivative (1b).
FIG. 20 is a graph showing the monoamine oxidase (MAO) inhibition efficiency (IC₅₀) of the synthesized cannabidiol derivative (1c).

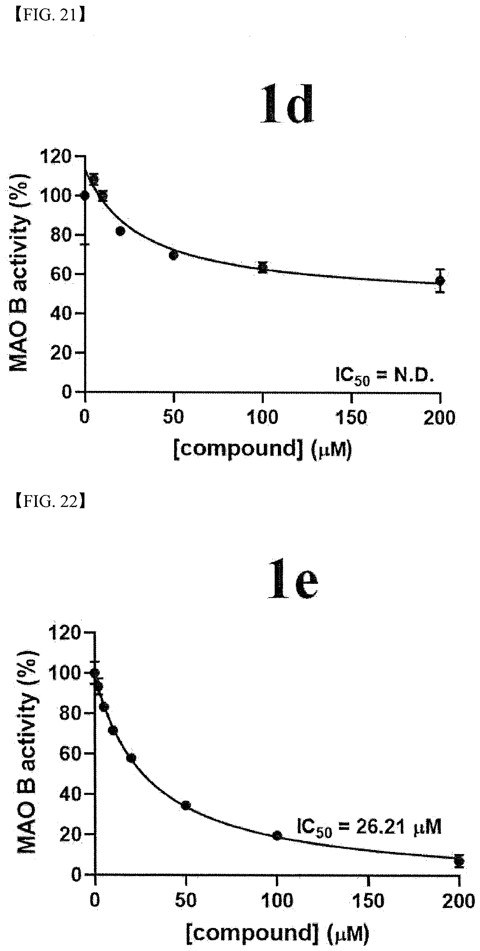

FIG. 21 is a graph showing the monoamine oxidase (MAO) inhibition efficiency (IC₅₀) of the synthesized cannabidiol derivative (1d).
FIG. 22 is a graph showing the monoamine oxidase (MAO) inhibition efficiency (IC₅₀) of the synthesized cannabidiol derivative (1e).

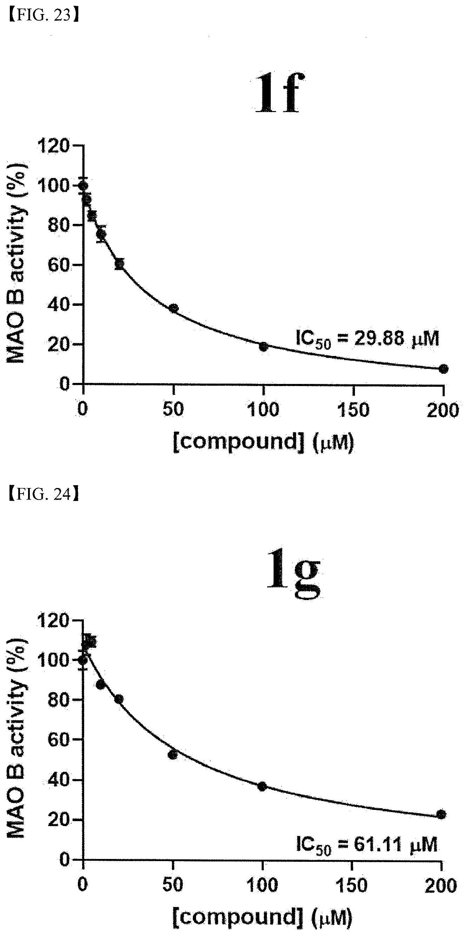

FIG. 23 is a graph showing the monoamine oxidase (MAO) inhibition efficiency (IC₅₀) of the synthesized cannabidiol derivative (1f).
FIG. 24 is a graph showing the monoamine oxidase (MAO) inhibition efficiency (IC₅₀) of the synthesized cannabidiol derivative (1g).

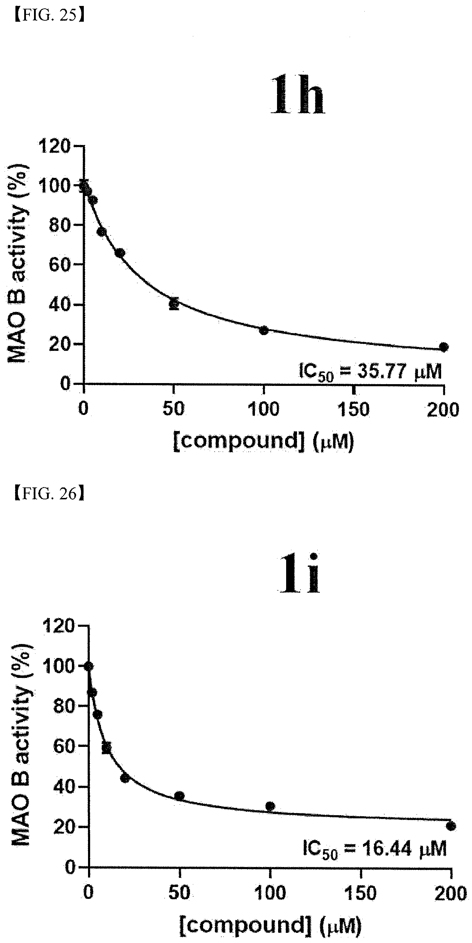

FIG. 25 is a graph showing the monoamine oxidase (MAO) inhibition efficiency (IC₅₀) of the synthesized cannabidiol derivative (1h).
FIG. 26 is a graph showing the monoamine oxidase (MAO) inhibition efficiency (IC₅₀) of the synthesized cannabidiol derivative (1i).

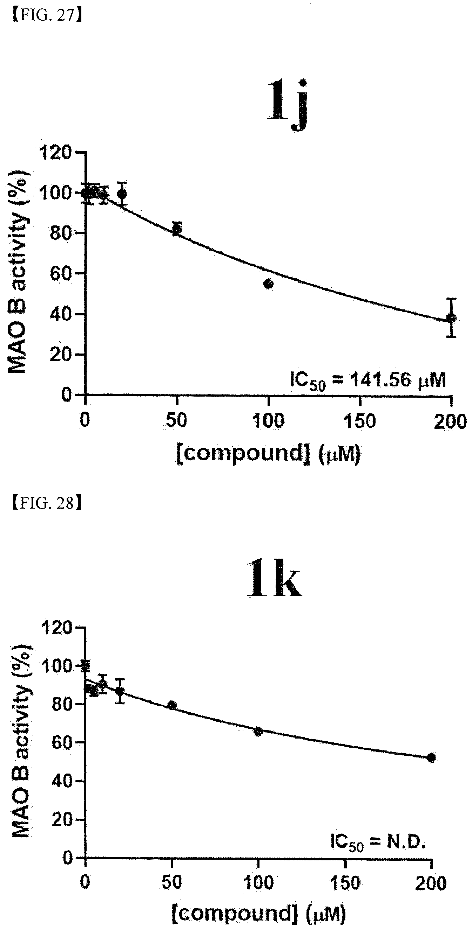

FIG. 27 is a graph showing the monoamine oxidase (MAO) inhibition efficiency (IC₅₀) of the synthesized cannabidiol derivative (1j).
FIG. 28 is a graph showing the monoamine oxidase (MAO) inhibition efficiency (IC₅₀) of the synthesized cannabidiol derivative (1k).

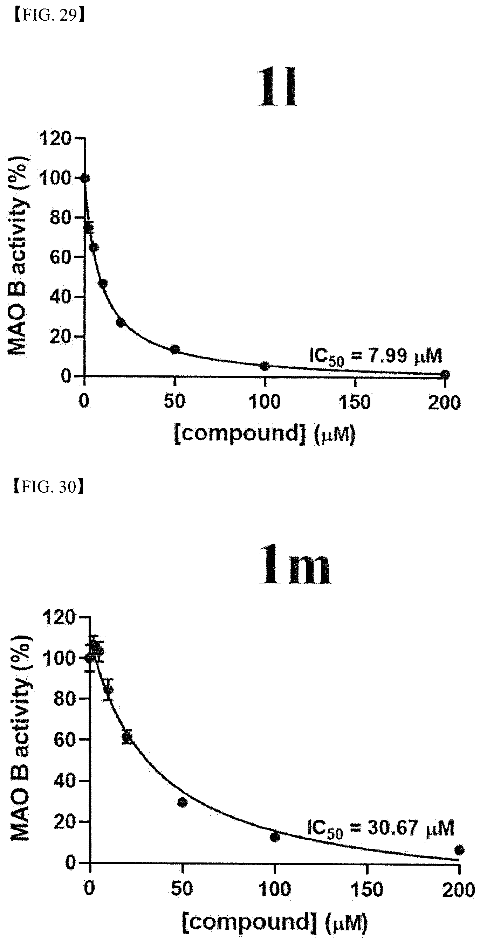

FIG. 29 is a graph showing the monoamine oxidase (MAO) inhibition efficiency (IC₅₀) of the synthesized cannabidiol derivative (11).
FIG. 30 is a graph showing the monoamine oxidase (MAO) inhibition efficiency (IC₅₀) of the synthesized cannabidiol derivative (1m).

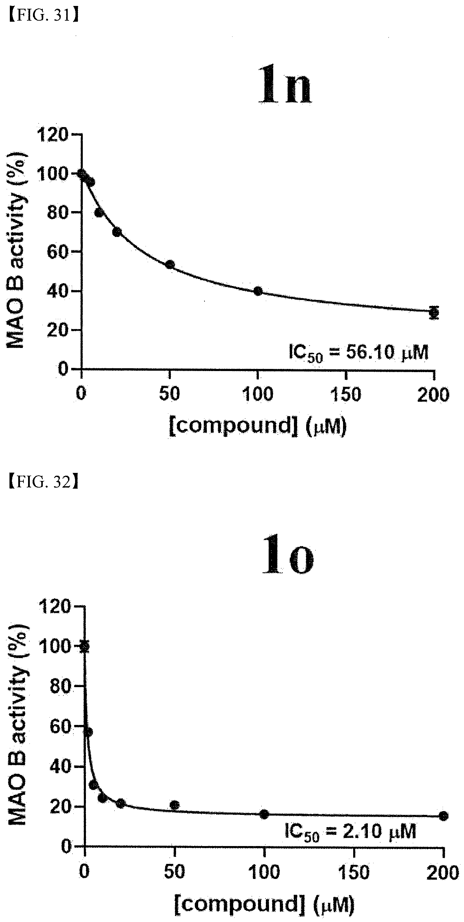

FIG. 31 is a graph showing the monoamine oxidase (MAO) inhibition efficiency (IC₅₀) of the synthesized cannabidiol derivative (1n).
FIG. 32 is a graph showing the monoamine oxidase (MAO) inhibition efficiency (IC₅₀) of the synthesized cannabidiol derivative (1o).

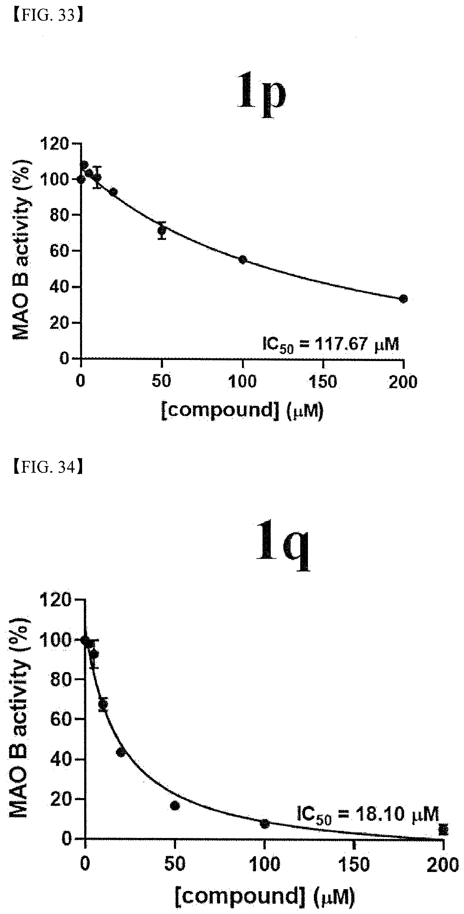

FIG. 33 is a graph showing the monoamine oxidase (MAO) inhibition efficiency (IC₅₀) of the synthesized cannabidiol derivative (1p).
FIG. 34 is a graph showing the monoamine oxidase (MAO) inhibition efficiency (IC₅₀) of the synthesized cannabidiol derivative (1q).

### DETAILED DESCRIPTION OF THE INVENTION

The present invention provides a cannabidiol derivative compound represented by the following Formula 1, a hydrate thereof, a solvate thereof or a pharmaceutically acceptable salt thereof, wherein: 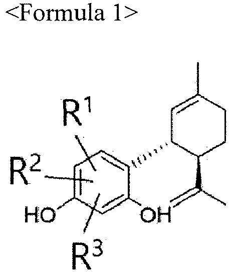
R¹, R², R³ are independently hydrogen, C₁-C₆ straight or branched chain alkyl, C₁-C₄ alkoxy, halogen, cyano, hydroxy, carboxyl, acetyl, dipentenyl, CONHR^{2a} or arylalkyl,
R^{2a} is arylalkyl substituted with hydroxy or alkoxy.

In one embodiment, the cannabidiol derivative compound represented by Formula 1 may be represented by Formula 1-1 below. 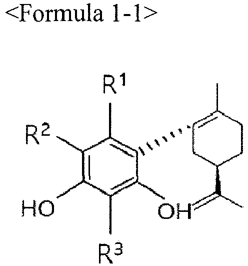 wherein R¹, R² or R³ is the same as defined above.

In one embodiment, R¹ may be hydrogen, methyl, propyl, pentyl, methoxy, bromo, cyano, hydroxy, acetyl or phenethyl.

In one embodiment, R² may be hydrogen, chloro, CONHR^{2a} or dipentenyl

In one embodiment, R^{2a} may be methoxyphenethyl or hydroxyphenethyl.

In one embodiment, R³ may be hydrogen, bromo, acetyl, carboxyl or dipentenyl.

Representative examples of cannabidiol derivative compounds according to the present invention are as follows:
[1](1'R,2'R)-5'-methyl-6-pentyl-2'-(prop-1-en-2-yl)-1',2',3',4'-tetrahydro-[1,1'-biphenyl]-2,4-diol (1a),
[2] (1'R,2'R)-5'-methyl-2'-(prop-1-en-2-yl)-6-propyl-1',2',3',4'-tetrahydro-[1,1'-biphenyl]-2,4-diol (1b),
[3] (1'R,2'R)-5',6-dimethyl-2'-(prop-1-en-2-yl)-1',2',3',4'-tetrahydro-[1,1'-biphenyl]-2,4-diol (1c),
[4] (1'R,2'R)-5'-methyl-6-phenethyl-2'-(prop-1-en-2-yl)-1',2',3',4'-tetrahydro-[1,1'-biphenyl]-2,4-diol (1d),
[5] (1'R,2'R)-6-bromo-5'-methyl-2'-(prop-1-en-2-yl)-1',2',3',4'-tetrahydro-[1,1'-biphenyl]-2,4-diol (1e),
[6] (1'R,2'R)-5-chloro-5'-methyl-2'-(prop-1-en-2-yl)-1',2',3',4'-tetrahydro-[1,1'-biphenyl]-2,4-diol (1f),
[7] (1'R,2'R)-4,6-dihydroxy-5'-methyl-2'-(prop-1-en-2-yl)-1',2',3',4'-tetrahydro-[1,1'-biphenyl]-2-carbonitrile (1g),
[8] (1'R,2'R)-6-methoxy-5'-methyl-2'-(prop-1-en-2-yl)-1',2',3',4'-tetrahydro-[1,1'-biphenyl]-2,4-diol (1h),
[9] 1-((1'R,2'R)-2,4-dihydroxy-5'-methyl-2'-(prop-1-en-2-yl)-1',2',3',4'-tetrahydro-[1,1'-biphenyl]-3-yl)ethan-1-one (1i),
[10] 1-((1R,1"R,2R,2"R)-2',4',6'-trihydroxy-5,5"-dimethyl-2,2"-di(prop-1-en-2-yl)-1,1",2,2",3,3",4,4"-octahydro-[1,1':3',1"-terphenyl]-5'-yl)ethan-1-one(1j),
[11] (1R,1"S,2R,2"S)-6'-methoxy-5,5"-dimethyl-2,2"-di(prop-1-en-2-yl)-1,1",2,2",3,3",4,4"-octahydro-[1,1':3',1"-terphenyl]-2',4'-diol(1k),
[12] 1-((1'R,2'R)-2,4,6-trihydroxy-5'-methyl-2'-(prop-1-en-2-yl)-1',2',3',4'-tetrahydro-[1,1'-biphenyl]-3-yl)ethan-1-one (11),
[13] (1'R,2'R)-3-bromo-5'-methyl-2'-(prop-1-en-2-yl)-1',2',3',4'-tetrahydro-[1,1'-biphenyl]-2,4-diol (1m),
[14] (1'R,2'R)-2,4-dihydroxy-5'-methyl-2'-(prop-1-en-2-yl)-1',2',3',4'-tetrahydro-[1,1'-biphenyl]-3-carboxylic acid (1n),
[15] (1'R,2'R)-4,6-dihydroxy-*N-*(4-methoxyphenethyl)-5'-methyl-2'-(prop-1-en-2-yl)-1',2',3',4'-tetrahydro-[1,1'-biphenyl]-3-carboxamide(1o),
[16] 1-((1'R,2'R)-4,6-dihydroxy-5'-methyl-2'-(prop-1-en-2-yl)-1',2',3',4'-tetrahydro-[1,1'-biphenyl]-2-yl)ethan-1-one (1p), 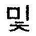
[17] (1'R,2'R)-4,6-dihydroxy-*N*-(4-hydroxyphenethyl)-5'-methyl-2'-(prop-1-en-2-yl)-1',2',3',4'-tetrahydro-[1,1'-biphenyl]-3-carboxamide(1q).

This specification uses the following definitions when defining the compound of Formula 1 unless specifically defined.

The term "alkyl" refers to a straight or branched chain hydrocarbonyl group, preferably C₁-C₁₀ alkyl. Examples of alkyl include, but are not limited to, methyl, ethyl, *n*-propyl, *iso*propyl, *n*-butyl, *iso*-butyl, *tert*-butyl, *n*-pentyl, *iso-*pentyl, *n*-hexyl, 3-methylhexyl, 2,2-dimethylpentyl, 2,3-dimethylpentyl, n-heptyl, n-octyl, n-nonyl and n-decyl.

The term "hydroxy" is defined as -OH, and the term "alkoxy" means alkyloxy, a radical in which the hydrogen atom of the hydroxy group is substituted by 1 to 10 alkyl, unless otherwise defined.

The term "carboxylic acid", "carboxyl" and "carboxy" refers to -COOH.

The term "cyano" refers to -CN group.

The term "acetyl" refers to -COCH₃.

The term "halogen" or "halo" refers to fluorine (F), chlorine (Cl), bromine (Br) or iodine (I).

The term "aryl" means aromatic hydrocarbon, includes a polycycle aromatic ring system in which a carbocycle aromatic ring or heteroaryl ring is fused with one or more other rings, preferably C₅-C₁₂ aryl, more preferably C₅-C₁₀ aryl. For example, aryl includes, but are not limited to, phenyl, naphthyl, tetrahydronaphthyl, etc.

Arylalkyl, alkylaryl and heteroarylalkyl refer to a group formed by combining aryl and alkyl or heteroaryl and alkyl as defined above, and include, for example, benzyl, phenethyl etc., but are not limited thereto.

The compound represented by Formula 1 according to the present invention can be prepared and used in the form of prodrugs, hydrates, solvates and pharmaceutically acceptable salts to enhance *in vivo* absorption or increase solubility, so the prodrugs, hydrates, solvates and pharmaceutically acceptable salts are also within the scope of the present invention.

The term "prodrug" refers to a substance that is transformed *in vivo* into a parent drug. Prodrugs are often used because, in some cases, they are easier to administer than the parent drug. For example, they may be bioavailable by oral administration, whereas the parent drug may not be. Prodrugs may also have improved solubility in pharmaceutical compositions than the parent drug. For example, a prodrug may be an *in vivo* hydrolysable ester of the compound according to the present invention and a pharmaceutically acceptable salt thereof. Another example of a prodrug may be a short peptide (poly-amino acid) in which the peptide is coupled to an acid group that is metabolically converted to reveal the active site.

The term "hydrate" refers to a compound of the present invention or a salt thereof containing a stoichiometric or non-stoichiometric amount of water bound by non-covalent intermolecular forces.

The term "solvate" refers to a compound of the present invention or a salt thereof containing a stoichiometric or non-stoichiometric amount of solvent bound by non-covalent intermolecular forces. Preferred solvents therefor include solvents that are volatile, non-toxic, and/or suitable for administration to humans.

The term "isomer" refers to a compound of the present invention or a salt thereof that has the same chemical formula or molecular formula but is structurally or sterically different. Such isomers include both structural isomer such as tautomer, and stereoisomers such as R or S isomers with asymmetric carbon center and geometric isomers (trans, cis). All of these isomers and their mixtures thereof are also included within the scope of the present invention.

The term "pharmaceutically acceptable salt" refers to a salt form of a compound that does not cause serious irritation to the organism to which the compound is administered and does not impair the biological activity and physical properties of the compound. The pharmaceutical salts include an acid addition salt formed by an acid containing a pharmaceutically acceptable anion and forming a non-toxic acid addition salt, for example, inorganic acids such as hydrochloric acid, sulfuric acid, nitric acid, phosphoric acid, hydrobromic acid, hydrogen iodide, etc., organic carbon acids such as tartaric acid, formic acid, citric acid, acetic acid, trichloroacetic acid, trifluoroacetic acid, gluconic acid, benzoic acid, lactic acid, fumaric acid, malic acid, salicylic acid, etc., sulfonic acids such as methanesulfonic acid, ethanesulfonic acid, benzenesulfonic acid, p-toluenesulfonic acid, etc. For example, pharmaceutically acceptable carboxylic acid salts include metal salts or alkaline earth metal salts formed by lithium, sodium, potassium, calcium, magnesium, etc., amino acid salts such as lysine, arginine, guanidine, etc., organic salts such as dicyclohexylamine, N-methyl-D-glucamine, tris(hydroxymethyl)methylamine, diethanolamine, choline and triethylamine etc. The compound of Formula 1 according to the present invention can also be converted into its salt by conventional methods.

In addition, the compound represented by the Formula 1 according to the present invention also includes hydrates and solvates that can be prepared therefrom.

The present invention also provides a pharmaceutical composition for cognitive function improvement, comprising the cannabidiol derivative compound represented by the Formula 1, a hydrate thereof, a solvate thereof or a pharmaceutically acceptable salt thereof, as an active ingredient.

The present invention also provides a pharmaceutical composition for cognitive function improvement, characterized in that the cognitive function improvement is preventing or treating degenerative brain disease.

In one embodiment, the degenerative brain disease may be one or more selected from the group consisting of Alzheimer's disease, mild cognitive impairment, stroke and vascular dementia, frontotemporal dementia, Lewy body dementia, Creutzfeldt-Jakob disease, traumatic head injury, syphilis, acquired immunodeficiency syndrome and other viral infections, brain abscess, brain tumor, multiple sclerosis, dementia caused by metabolic diseases, hypoxia, Parkinson's disease, Lou Gehrig's disease, Huntington's disease, Pick's disease, amyotrophic lateral sclerosis, epilepsy, ischemia, stroke, attention deficit hyperactivity disorder, schizophrenia, depression, bipolar disorder, post-traumatic stress disorder, spinal cord injury, and myelitis.

The compounds represented by the Formula 1, and pharmaceutically acceptable salts thereof, show excellent acetylcholinesterase, butyrylcholinesterase and monoamine oxidase inhibitory activity, and thus can be used for cognitive function improvement, and in particular, can be useful in the treatment or prevention of dementia. Accordingly, the compounds of the present invention can simultaneously improve the decrease in acetylcholine levels in brain tissue and the decrease in blood butyrylcholine levels in dementia patients, thereby reducing the dosage of each drug and thus reducing the side effects of dementia treatment.

The additive may include a pharmaceutically acceptable carrier or diluent, each of which may be formulated according to conventional methods in the form of oral formulations such as powders, granules, tablets, capsules, suspensions, emulsions, syrups, aerosols; topicals; suppositories; and sterile injectable solutions. The pharmaceutically acceptable carriers include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate and mineral oil, and the like. They also include diluents or excipients such as fillers, bulking agents, binders, wetting agents, disintegrating agents, and surfactants. Oral solid dosage forms include tablets, pills, powders, granules, capsules, and the like, which may include at least one excipient, such as starch, calcium carbonate, sucrose, lactose, gelatin, and the like, and may include lubricants such as magnesium stearate and talc. Oral liquid preparations may include suspensions, oral solutions, emulsions, syrups, and the like, and may include diluents such as water and liquid paraffin, wetting agents, sweeteners, flavourings, preservatives, and the like. Parenteral preparations include sterilized aqueous solutions, non-aqueous solvents, suspensions, emulsions, creams, lyophilised preparations, and suppositories; non-aqueous solvents and suspensions include propylene glycol, polyethylene glycol, vegetable oils such as olive oil, and injectable esters such as ethylolate. Substrates for suppositories may be witepsol, macrogol, tween 61, cacao gum, laurin gum, glycerogelatin, etc.

The pharmaceutical compositions of the present invention can be administered to mammals, such as livestock and humans, by various routes, e.g., by oral, dermal, subcutaneous, intramuscular, intravenous, intraperitoneal, rectal, intrauterine, intrathecal or cerebrovascular injection, and topical administration. Accordingly, the compositions of the present invention can be formulated in various forms, such as tablets, capsules, aqueous solutions or suspensions. For oral tablets, a carrier such as lactose, cornstarch, or the like, and a disintegrating agent such as magnesium stearate may be typically added. For oral capsules, lactose and/or dry cornstarch may be used as diluents. If an oral aqueous suspension is desired, the active ingredient may be combined with an emulsifier and/or suspending agent. If necessary, specific sweeteners and/or flavoring agents may be added. For intramuscular, intraperitoneal, subcutaneous and intravenous administration, a sterile solution of the active ingredient is usually prepared, and the pH of the solution should be adjusted and buffered accordingly. For intravenous administration, the total concentration of the solute should be adjusted to impart isotonicity to the preparation. The composition according to the invention may be in the form of an aqueous solution containing a pharmaceutically acceptable carrier, such as brine with a pH of 7.4. The solution may be introduced into the intramuscular bloodstream of a patient by local injection.

The dosage of the active ingredient in the pharmaceutical composition of the present invention depends on the condition and weight of the patient, the extent of the disease, the formulation of the active ingredient, the route and duration of administration, and may be appropriately adjusted depending on the patient. For example, the active ingredient can be administered at a dose of 0.0001 to 1000 mg/kg per day, preferably 0.01 to 100 mg/kg, and the dose may be administered once or in several divided doses per day. Furthermore, the pharmaceutical composition of the present invention may comprise the active ingredient from 0.001 to 90% by weight, based on the total weight of the composition.

The present invention also provides a composition for inhibiting monoamine oxidase (MAO) comprising the cannabidiol derivative compound represented by the above Formula 1, a hydrate thereof, a solvate thereof or a pharmaceutically acceptable salt thereof as an active ingredient.

The present invention also provides a food composition for cognitive function improvement comprising the cannabidiol derivative compound represented by the above Formula 1, a hydrate thereof, a solvate thereof or a pharmaceutically acceptable salt thereof as an active ingredient.

The food composition of the present invention can be used as a health functional food. The term "health functional food" refers to food manufactured and processed using raw materials or ingredients that have functional properties useful to the human body in accordance with the Act on Health Functional Foods, and the term "functional" refers to food consumed for the purpose of obtaining effects useful for health purposes such as nutrient regulation or physiological action on the structure and function of the human body.

The food compositions of the present invention may contain conventional food additives, and their suitability as "food additives" shall be determined according to the standards and criteria for such items in accordance with the general rules and general test methods for Korea Food Additives Code approved by the Ministry of Food and Drug Safety, unless otherwise specified.

The items listed in the "Korea Food Additives Code" above include, for example, chemical compounds such as ketones, glycine, potassium citrate, nicotinic acid, and cinnamic acid; natural additives such as persimmon coloring, licorice extract, crystalline cellulose, macrocycles, and guar gum; monosodium L-glutamate preparations; and mixed preparations such as noodle alkalis, preservatives, and tar colorants.

The food compositions of the present invention may comprise from 0.01 to 95%, preferably from 1 to 80% by weight, of the compound represented by Formula 1, relative to the total weight of the composition, for the purpose of improving cognitive function, in particular for the prevention and/or improvement of dementia, depression, Parkinson's disease. It can also be prepared and processed in the form of tablets, capsules, powders, granules, liquids, pills, etc. for the purpose of improving cognitive function, in particular for the prevention and/or improvement of dementia, depression, Parkinson's disease.

The present invention also provides a method for preparing a cannabidiol derivative compound represented by the following Formula 1 , comprising: obtaining a first mixture by mixing a benzoic acid compound represented by Formula 2 below and cis-isolimonenol represented by Formula 3 below; reacting the obtained first mixture with p-toluenesulfinic acid or oxalic acid hydrate to obtain a second mixture comprising a compound represented by Formula 4 below and a compound represented by Formula 1 below; and purifying the obtained second mixture to obtain a cannabidiol derivative compound represented by Formula 1 below. 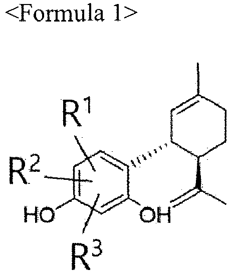 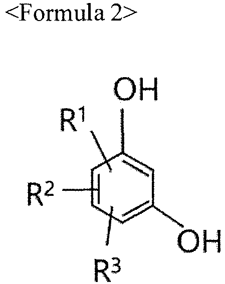 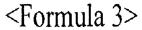 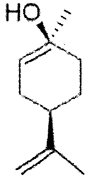 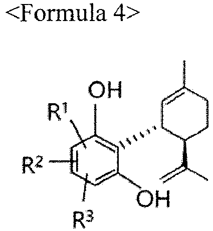 wherein in Formula 1, Formula 2, Formula 3 and Formula 4, R¹, R² and R³ are the same as defined above.

Reaction 1 of the Synthesis Example is exemplified as a process for synthesizing the compounds of Formula 1 of the present invention, and the method of preparation of Reaction 1 is not intended to be limiting to methods for preparing the compounds of Formula 1 according to the present invention. It will be appreciated that the method of preparation of Formula 1 is exemplary only and can be readily modified by one of ordinary skill in the art depending on specific substituents.

Hereinafter, the present invention will be described in more detail with Synthesis Examples, Examples and Experimental examples. However, the following Synthesis Examples, Examples and Experimental examples are intended to illustrate the present invention and are not intended to limit the scope of the present invention.

### Synthesis Example 1. General synthesis method of cannabidiol derivative compound

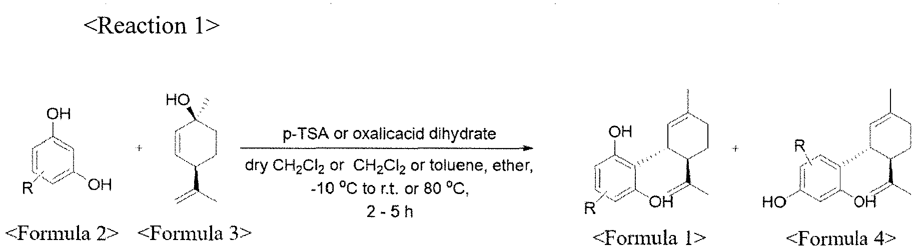

A first mixture was obtained by mixing a benzoic acid compound and cis-isolimonenol in a round flask, and p-toluenesulfinic acid or oxalic acid hydrate was added to the mixture at - 10°C, dissolved in methane dichloride (5 ml), and then added dropwise. Stir at room temperature to obtain a second mixture. The reaction was terminated by adding an aqueous solution of sodium bicarbonate to the second mixture, and the mixture was extracted three times with diethyl ether. The extracted organic layer was dried using magnesium sulfate, and The residue was purified through column chromatography to obtain a compound.

### Example 1. (1'R,2'R)-5'-methyl-6-pentyl-2'-(prop-1-en-2-yl)-1',2',3',4'-tetrahydro-[1,1'-biphenyl]-2,4-diol (compound 1a)

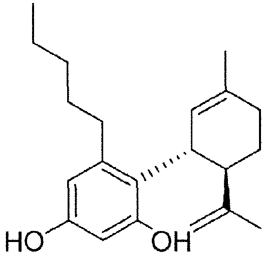

Olivetol (3.0 mmol, 540.7 mg) and anhydrous dichloromethane (5.0 mL) were added and dissolved in a 100 mL round flask under nitrogen substitution. P-toluenesulfinic acid (0.3 mmol, 57.0 mg) was added at -10°C and (1S,4R)-1-methyl-4-(prop-1-en-2-yl)cyclohex-2-en-1-ol (3.3 mmol, 0.53 ml) was dissolved in methane dichloride (5 mL), added dropwise, and stirred at room temperature for 3 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1a.** (Silica gel, ethyl acetate/hexane = 1:50, v/v) (brown gum, 399.8 mg, 42% yield).

¹H NMR (400 MHz, CDCl₃-*d*₁) δ 6.21-6.19 (m, 2H), 6.05 (s, 1H), 5.52 (s, 1H), 4.65-4.64 (m, 1H), 4.57 (s, 1H), 4.46 (s, 1H), 3.54-3.52 (m, 1H), 2.62-2.55 (dd, 1H), 2.51-2.44 (m, 1H), 2.29-2.20 (m, 2H), 2.12-2.05 (m, 1H), 1.84-1.75 (m, 5H), 1.53 (s, 3H), 1.48-1.45 (m, 2H), 1.33-1.30 (m, 4H), 0.91-0.87 (m, 3H).

### Example 2. (1'R,2'R)-5'-methyl-2'-(prop-1-en-2-yl)-6-propyl-1',2',3',4'-tetrahydro-[1,1'-biphenyl]-2,4-diol (compound 1b)

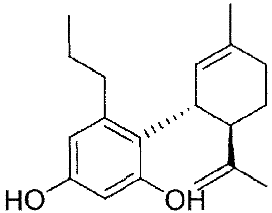

5-Propylbenzene-1,3-diol (3.0 mmol, 456.6 mg) and anhydrous dichloromethane (5.0 mL) were added and dissolved in a 100 mL round flask under nitrogen substitution. P-Toluenesulfinic acid (0.3 mmol, 57.0 mg) was added at -10°C, and (1S,4R)-1-methyl-4-(prop-1-en-2-yl)cyclohex-2-en-1-ol (3.3 mmol, 0.53 ml) was dissolved in methane dichloride (10 mL), added dropwise, and stirred at room temperature for 2 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1**b. (Silica gel, ethyl acetate/hexane = 1:50, v/v) (brown gum, 467.7 mg, 54% yield).

¹H NMR (400 MHz, CDCl₃-*d*₁) δ 6.21-6.19 (m, 2H), 6.04 (m, 1H), 5.52 (s, 1H), 4.65-4.64 (m, 1H), 4.46 (s, 1H), 3.54-3.52 (m, 1H), 2.62-2.55 (m, 1H), 2.51-5.45 (m, 1H), 2.28-2.20 (m, 2H), 2.21-2.11 (m, 1H), 1.83-1.79 (m, 5H), 1.53 (s, 3H), 1.51-1.46 (m, 2H), 0.92 (t, J = 7.32 Hz, 3H).

### Example 3. (1'R,2'R)-5',6-dimethyl-2'-(prop-1-en-2-yl)-1',2',3',4'- tetrahydro-[1,1'-biphenyl]-2,4-diol (compound 1c)

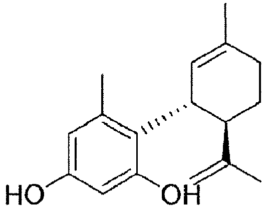

Orciol (3.0 mmol, 372.42 mg) and anhydrous dichloromethane (10.0 mL) were added to a 100 mL round flask under nitrogen substitution and stirred at room temperature until dissolved. P-Toluenesulfinic acid (0.3 mmol, 57.0 mg) was added at -10°C, and (1S,4R)-1-methyl-4-(prop-1-en-2-yl)cyclohex-2-en- 1-ol (3.3 mmol, 0.53 ml) was dissolved in methane dichloride (5 mL), added dropwise, and stirred at room temperature for 3 hours and 30 minutes. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1c**. (Silica gel, ethyl acetate/hexane = 1:50, v/v) (brown gum, 256.1 mg, 33% yield).

¹H NMR (400 MHz, CDCl₃-*d*₁) δ 6.21-6.19 (m, 2H), 6.07 (s, 1H), 5.54 (s, 1H), 4.65-4.65 (m, 1H), 4.58 (s, 1H), 4.46 (s, 1H), 3.56-3.54 (m, 1H), 2.48-2.42 (m, 1H), 2.26-2.19 (m, 1H), 2.15 (s, 3H), 2.12-2.07(m, 1H) 1.83-1.79 (m, 5H), 1.57 (s, 3H).

### Example 4. (1'R,2'R)-5'-methyl-6-phenethyl-2'-(prop-1-en-2-yl)-1',2',3',4'-tetrahydro-[1,1'-biphenyl]-2,4-diol (compound 1d)

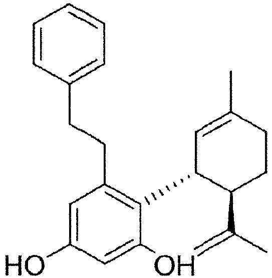

5-Phenethylbenzene-1,3-diol (2.0 mmol, 428.5 mg) and methane dichloride (6.6 mL) were added and dissolved in a 100 mL round flask. P-toluenesulfinic acid (0.2 mmol, 38.0 mg) was added at -10°C and (1S,4R)-1-methyl-4-(prop-1-en-2-yl)cyclohex-2-en-1-ol (2.2 mmol, 0.35 ml) was dissolved in methane dichloride (6.6 mL), added dropwise, and stirred at room temperature for 2 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1d**. (Silica gel, ethyl acetate/hexane = 1:50, v/v) (brown oil, 72.8 mg, 11 % yield).

¹H NMR (400 MHz, CDCl₃-*d*₁) δ 7.30 - 7.27(m, 2H), 7.23 - 7.14(m, 3H), 6.24(d, *J* = 2.6, 1H), 6.22(d, *J* = 2.6, 1H), 6.08(s, 1H), 5.41(s,1H), 4.66(t, *J* = 1.5, 1H), 4.47(s, 1H), 3.56 - 3.53(m, 1H), 2.91 - 2.86(m, 1H), 2.78(t, J = 8.0, 2H), 2.64 - 2.59(m, 1H), 2.50 - 2.44(m, 1H), 2.22 - 2.11(m, 1H), 1.84 - 1.68(m, 5H), 1.58 - 1.56(m, 1H), 1.52(s, 3H), 1.30(d, *J* = 6.8, 1H).

### Example 5. (1'R,2'R)-5',6-dimethyl-2'-(prop-1-en-2-yl)-1',2',3',4'- tetrahydro-[1,1'-(1'R,2'R)-6-bromo-5'-methyl-2'-(prop-1-en-2-yl)-1',2',3',4'-tetrahydro-[1,1'-biphenyl]-2,4-diol (compound 1e)

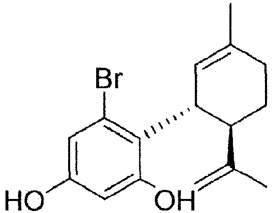

5-Bromoresorcinol (1.0 mmol, 189.01 mg) and anhydrous dichloromethane (10.0 mL) were added and dissolved in a 100 mL round flask under nitrogen substitution. P-toluenesulfinic acid (0.1 mmol, 19.0 mg) was added at -10°C and (1S,4R)-1-methyl-4-(prop-1-en-2-yl)cyclohex-2-en-1-ol (1.0 mmol, 0.16 ml) was dissolved in methane dichloride (6.6 mL), added dropwise, and stirred at room temperature for 3 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1e**. (Silica gel, ethyl acetate/hexane = 1:50, v/v) (brown oil, 72.8 mg, 11 % yield).

¹H NMR (400 MHz, CDCl₃-*d*₁) δ 6.62 (d, *J* = 2.60 Hz, 1H), 6.31 (d, *J* = 2.58 Hz, 1H), 6.27 (s, 1H), 5.55 (s, 1H), 4.77 - 4.73 (m, 1H), 4.62 - 4.61 (m, 1H), 4.37 - 4.36 (m, 1H), 3.98 - 3.94 (m, 1H), 2.52 - 2.46 (m, 1H), 2.22 - 2.06 (m, 2H), 1.82 - 1.69 (m, 8H).

### Example 6. (1'R,2'R)-5-chloro-5'-methyl-2'-(prop-1-en-2-yl)-1',2',3',4'-tetrahydro-[1,1'-biphenyl]-2,4-diol (compound 1f)

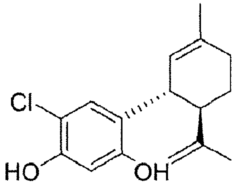

4-Chlororesorcinol (0.7 mmol, 100.0 mg) and toluene (10 mL) were added to a 100 mL round flask and stirred at room temperature until dissolved. P-toluenesulfinic acid (0.28 mmol, 53.26 mg) was added at -10°C and (1S,4R)-1-methyl-4-(prop-1-en-2-yl)cyclohex-2-en-1-ol (0.91 mmol, 0.15 ml) was dissolved in toluene (1 mL), added dropwise, and stirred at room temperature for 4 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1f**. (Silica gel, ethyl acetate/hexane = 1:50, v/v) (yellow oil, 21.4 mg, 11 % yield).

¹H NMR (400 MHz, CDCl₃-*d*₁) δ 6.89(s, 1H), 6.49(s, 1H), 5.44(s, 1H), 4.71 - 4.70 (m, 1H), 4.57 - 4.56 (m, 1H), 3.36 - 3.32 (m, 1H), 2.27 - 2.14 (m, 2H), 2.08 - 2.03(m, 1H), 1.83 - 1.69(m, 7 H), 1.61 - 1.60(m, 3H).

### Example 7. (1'R,2'R)-4,6-dihydroxy-5'-methyl-2'-(prop-1-en-2-yl)-1',2',3',4'-tetrahydro-[1,1'-biphenyl]-2-carbonitrile (compound 1g)

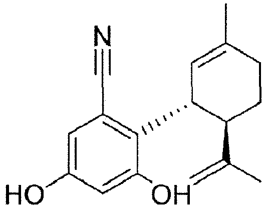

3,5-Dihydroxybenzonitrile (0.74 mmol, 100.0 mg) and toluene:ether = 2:1 (12 mL) were added to a 100 mL round flask and stirred at room temperature until dissolved. Oxalic acid hydrate (0.74 mmol, 93.3 mg) was added at -10°C and (1S,4R)-1-methyl-4-(prop-1-en-2-yl)cyclohex-2-en-1-ol( 0.81 mmol, 0.13 ml) was dissolved in toluene (1 mL), added dropwise, and stirred at room temperature for 4 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1g.** (Silica gel, ethyl acetate/hexane = 1:50, v/v) (brown oil, 12.0 mg, 6 % yield).

¹H NMR (400 MHz, CDCl₃-*d*₁) δ 6.64(d, *J* = 2.48, 1H), 6.57(d, *J* = 2.56, 1H), 6.23(s, 1H), 5.55(s, 1H), 4.66 (s, 1H), 4.34 (s, 1H), 3.80 - 3.77 (m, 1H), 2.43 (t, *J* = 9.52, 1H), 2.25 - 2.22(m, 1H), 2.15 - 2.11(m, 1H), 1.86 - 1.74(m, 6H), 1.70(m, 3H).

### Example 8. (1'R,2'R)-6-methoxy-5'-methyl-2'-(prop-1-en-2-yl)-1',2',3',4'-tetrahydro-[1,1'-biphenyl]-2,4-diol (compound 1h)

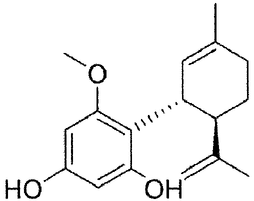

5-Methoxyresorcinol (0.72 mmol, 100.9 mg) and toluene:ether = 4:1 (10 mL) were added to a 100 mL round flask and stirred at room temperature until dissolved. P-toluenesulfinic acid (0.29 mmol, 54.78 mg) was added at -10°C and (1S,4R)-1-methyl-4-(prop-1-en-2-yl)cyclohex-2-en-1-ol (0.79 mmol, 0.13 ml) was dissolved in toluene (1 mL), added dropwise, and stirred at room temperature for 2 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1h**. (Silica gel, ethyl acetate/hexane = 1:30, v/v) (brown oil, 35.1 mg, 18 % yield).

1H NMR (400 MHz, CDCl₃-*d*₁) δ 6.11(s, 1H), 5.96 - 5.95(m, 1H), 5.55(s, 1H), 4.50 - 4.49(m, 1H), 4.32 (s, 1H), 3.94 - 3.92 (m, 1H), 3.67 (s, 3H), 2.38 -2.32 (m, 1H), 2.21 - 2.22(m, 1H), 2.10 - 2.08(m, 1H), 1.79 - 1.72(m, 6H), 1.65(s, 3H).

### Example 9. 1-((1'R,2'R)-2,4-dihydroxy-5'-methyl-2'-(prop-1-en-2-yl)-1',2',3',4'-tetrahydro-[1,1'-biphenyl]-3-yl)ethan-1-one (compound 1i)

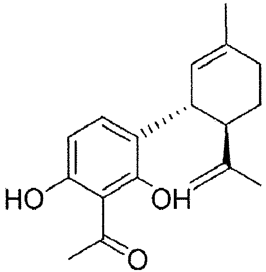

2',6'-Dihydroxyacetophenone (2.0 mmol, 304.3 mg) and toluene:ether = 4:1 (30 mL) were added to a 100 mL round flask and stirred at room temperature until dissolved. P-toluenesulfinic acid (0.8 mmol, 152.18 mg) was added at -10°C and (1S,4R)-1-methyl-4-(prop-1-en-2-yl)cyclohex-2-en-1-ol (2.2 mmol, 0.36 ml) was dissolved in toluene (3 mL), added dropwise, and stirred at room temperature for 4 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1i**. (Silica gel, ethyl acetate/hexane = 1:50, v/v) (brown oil, 36.4 mg, 6 % yield).

¹H NMR (400 MHz, CDCl₃-*d*₁) δ 7.02(d, *J* = 8.42, 1H), 6.33(d, *J* = 8.41, 1H), 5.45(s, 1H), 4.67 -4.66(m, 1H), 4.54 (s, 1H), 3.48 (s, 1H), 2.71 (s, 3H), 2.31 -2.25 (m, 1H), 2.21 - 2.18(m, 1H), 2.10 - 2.08(m, 1H), 1.79 - 1.72(m, 6H), 1.61(s, 3H).

### Example 10. 1-((1R,1"R,2R,2"R)-2',4',6'-trihydroxy-5,5"- dimethyl-2,2"-di(prop-1-en-2-yl)-1,1",2,2",3,3",4,4"-octahydro-[1,1':3',1"- terphenyl]-5'-yl)ethan-1-one (compound 1j)

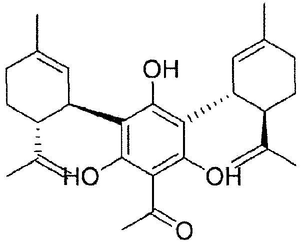

2,4,6-Trihydroxyacetophenone (1.79 mmol, 300.9 mg) and toluene:ether = 1:1 (48 mL) were added to a 100 mL round flask and stirred at room temperature until dissolved.

P-toluenesulfinic acid (0.72 mmol, 136.20 mg) was added at -10°C and (1S,4R)-1-methyl-4-(prop-1-en-2-yl)cyclohex-2-en-1-ol (1.97 mmol, 0.32 ml) was dissolved in toluene (3 mL), added dropwise, and stirred at room temperature for 5 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1j**. (Silica gel, ethyl acetate/hexane = 1:50, v/v) (yellow oil, 149.2 mg, 19 % yield).

¹H NMR (400 MHz, CDCl₃-*d*₁) δ 13.95(s, 1H), 6.77(s, 1H), 6.65(s, 1H), 5.55(s, 1H), 5.49(s, 1H), 4.56(s, 1H), 4.48(s, 1H), 4.44(s, 1H), 4.35(s, 1H), 4.07 - 4.04(m, 1H), 3.97 - 3.94(m, 1H), 2.63 (s, 3H), 2.40 - 2.39 (m, 1H), 2.29 - 2.23(m, 3H), 2.12 - 2.11(m, 2H), 1.82 - 1.73(m, 13H), 1.68 (s, 7H).

### Example 11. (1R,1"S,2R,2"S)-6'-methoxy-5,5"-dimethyl-2,2"-di(prop-1-en-2-yl)-1,1",2,2",3,3",4,4"-octahydro-[1,1':3',1"-terphenyl]-2',4'-diol (compound 1k)

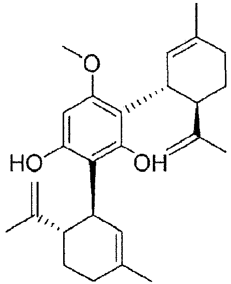

5-Methoxyresorcinol (0.72 mmol, 100.9 mg) and toluene:ether = 4:1 (10 mL) were added to a 100 mL round flask and stirred at room temperature until dissolved. P-toluenesulfinic acid (0.29 mmol, 54.78 mg) was added at -10°C and (1S,4R)-1-methyl-4-(prop-1-en-2-yl)cyclohex-2-en-1-ol (0.79 mmol, 0.13 ml) was dissolved in toluene (1 mL), added dropwise, and stirred at room temperature for 2 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1k.** (Silica gel, ethyl acetate/hexane = 1:50, v/v) (brown oil, 98.8 mg, 34 % yield).

¹H NMR (400 MHz, CDCl₃-*d*₁) δ 6.00(s, 1H), 5.95(s, 1H), 5.91(s, 1H), 5.56(s, 1H), 5.52(s, 1H), 4.54(s, 1H), 4.45 - 4.44(m, 2H), 4.26(s, 1H), 3.92(s, 2H), 3.64 (s, 3H), 2.43 - 2.27 (m, 2H), 2.22 - 2.17(m, 2H), 2.10 - 2.08(m, 2H), 1.81 - 1.62(m, 20H).

### Example 12. 1-((1'R,2'R)-2,4,6-trihydroxy-5'-methyl-2'-(prop-1-en-2-yl)-1',2',3',4'-tetrahydro-[1,1'-biphenyl]-3-yl)ethan-1-one (compound 1l)

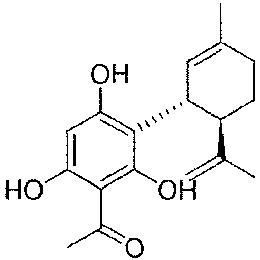

2,4,6-Trihydroxyacetophenone (1.79 mmol, 300.9 mg) and toluene:ether = 1:1 (48 mL) were added to a 100 mL round flask and stirred at room temperature until dissolved. P-toluenesulfinic acid (0.72 mmol, 136.20 mg) was added at -10°C and (1S,4R)-1-methyl-4-(prop-1-en-2-yl)cyclohex-2-en-1-ol (1.97 mmol, 0.32 ml) was dissolved in toluene (3 mL), added dropwise, and stirred at room temperature for 5 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **11.** (Silica gel, ethyl acetate/hexane = 1:50, v/v) (brown oil, 76.8 mg, 14 % yield).

¹H NMR (400 MHz, MeOD-*d*₄) δ 5.85(s, 1H), 5.21(s, 1H), 4.48 -4.46(m, 2H), 3.91 - 3.89(m, 1H), 2.98 - 2.97 (m, 1H), 2.61 (s, 3H), 2.19 -2.18 (m, 1H), 2.01 - 1.98(m, 1H), 1.77 - 1.71(m, 2H), 1.68 - 1.65(m, 8H).

### Example 13. (1'R,2'R)-3-bromo-5'-methyl-2'-(prop-1-en-2-yl)-1',2',3',4'-tetrahydro-[1,1'-biphenyl]-2,4-diol (compound 1m)

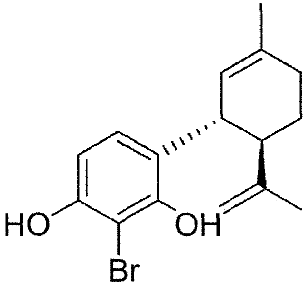

2-Bromoresorcinol (1.59 mmol, 300.0 mg) and toluene (30 mL) were added to a 100 mL round flask and stirred at room temperature until dissolved. P-toluenesulfinic acid (0.64 mmol, 120.98 mg) was added at -10°C and (1S,4R)-1-methyl-4-(prop-1-en-2-yl)cyclohex-2-en-1 -ol (1.75 mmol, 0.28 ml) was dissolved in toluene (3 mL), added dropwise, and stirred at room temperature for 2 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1m.** (Silica gel, ethyl acetate/hexane = 1:50, v/v) (yellow oil, 137.2 mg, 27% yield).

¹H NMR (400 MHz, CDCl₃-*d*₁) δ 6.87(d, *J* = 8.41, 1H), 6.53(d, *J* = 8.40, 1H), 5.89(s, 1H), 5.40(s, 1H), 4.64 - 4.63(m, 1H), 4.52 - 4.51 (m, 1H), 3.56 - 3.54(m, 1H), 2.32 -2.26 (m, 1H), 2.17 - 2.14(m, 1H), 2.06 - 2.01(m, 1H), 1.80 - 1.71(m, 6H), 1.62(s, 3H).

### Example 14. (1'R,2'R)-2,4-dihydroxy-5'-methyl-2'-(prop-1-en-2-yl)-1',2',3',4'-tetrahydro-[1,1'-biphenyl]-3-carboxylic acid (compound 1n)

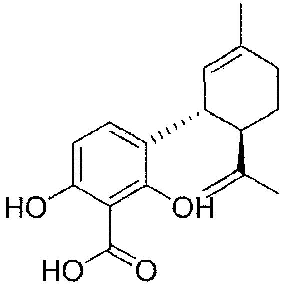

2,6-Dihydroxybenzoic acid (2.0 mmol, 308.24 mg) was added to a 100 mL round flask, and toluene:ether = 15:1 (32 mL) was added, stirred at room temperature until dissolved. P-toluenesulfinic acid (0.8 mmol, 152.18 mg) was added at -10°C and (1S,4R)-1-methyl-4-(prop-1-en-2-yl)cyclohex-2-ene-1-ol (2.2 mmol, 353.65 ul) was dissolved in toluene (2 mL), added dropwise, and stirred at room temperature for 2 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1n.** (Silica gel, Methanol/dichloromethane = 1:7, v/v) (brown oil, 10.2 mg, 7 % yield).

¹H NMR (400 MHz, MeOD-*d*₄) δ 6.93 (d, *J* = 8.45 Hz, 1H), 6.19 (d, *J* = 8.43 Hz, 1H), 5.23 (br s, 1H), 4.55 - 4.54 (m, 1H), 4.52-4.51 (m, 1H), 3.79 - 3.73 (m, 1H), 2.33 - 2.27 (m, 1H), 2.17 - 2.12 (m, 1H), 2.05 - 1.98 (m, 1H), 1.78 - 1.72 (m, 2H), 1.70 (br s, 3H), 1.68 (br s, 3H).

### Example 15. (1'R,2'R)-4,6-dihydroxy-N-(4-methoxyphenethyl)-5'-methyl-2'-(prop-1-en-2-yl)-1',2',3',4'-tetrahydro-[1,1'-biphenyl]-3-carboxamide (compound 1o)

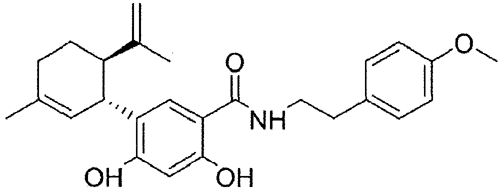

2,4-Dihydroxy-N-(4-methoxyphenethyl) benzamide (1.14 mmol, 327.54 mg) was added to a 100 mL round flask, and toluene:ether = 3:1 (20 mL) was added. After stirring until the mixture was dissolved, p-toluenesulfinic acid (0.46 mmol, 79.2 mg) was add at -10°C and (1S,4R)-1-methyl-4-(prop-1-en-2-yl) ) Cyclohex-2-en-1-ol (1.25 mmol, 203.09 ul) was dissolved in toluene (2 mL), added dropwise, and stirred at room temperature for 2 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1o**. (Silica gel, methanol/dichloromethane = 1:20, v/v) (yellow oil, 98 mg, 21% yield).

¹H NMR (400 MHz, MeOD-*d*₄) δ 7.40 (s, 1H), 7.17 (dd, *J* = 6.64, 2.00 Hz, 2H), 6.86 (dd, *J* = 6.64, 2.00 Hz, 2H), 6.25 (s, 1H), 5.22 (br s, 1H), 4.55-4.54 (m, 1H), 4.50-4.49 (m, 1H), 3.78 (s, 3H), 3.53 (dt, J = 7.20, 1.92 Hz, 2H), 2.84 (t, J = 7.28 Hz, 2H), 2.43 - 2.37 (m, 1H), 2.22 - 2.17 (m, 1H), 2.04 - 1.99 (m, 1H), 1.78 - 1.71 (m, 3H), 1.70 (br s, 3H), 1.68 (br s, 3H).

### Example 16. 1-((1'R,2'R)-4,6-dihydroxy-5'-methyl-2'-(prop-1-en-2-yl)-1',2',3',4'-tetrahydro-[1,1'-biphenyl]-2-yl)ethan-1-one (compound 1p)

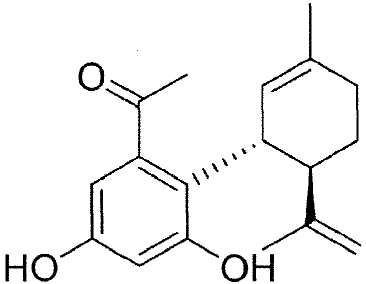

1-(3,5-Dihydroxyphenyl)ethan-1-one (3 mmol, 465.45 mg) was added to a 100 mL round flask, and toluene:ether = 3:1 (55 mL) was added. After stirring until the mixture was dissolved, p-toluenesulfinic acid (1.2 mmol, 206.64 mg) was added at -10°C and (1S,4R)-1-methyl-4-(prop-1-en-2-yl)cyclohex-2-en-1-ol (3.3 mmol, 502.43 ul) was dissolved in 2 mL of toluene, added dropwise, and stirred at room temperature for 2 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1p**. (Silica gel, methanol/dichloromethane = 1:15, v/v) (yellow crystals, 18 mg, 23% yield).

¹H NMR (400 MHz, MeOD-*d*₄) δ 6.32 (d, *J* = 2.08 Hz, 1H), 6.08 (d, *J* = 2.06 Hz, 1H), 5.59 (br s, 1H), 4.90 (d, *J* = 1.81 Hz, 1H), 3.39 - 3.36 (m, 1H), 2.59 - 2.48 (m, 1H), 2.24 - 2.22 (m, 1H), 2.20 (br s, 3H), 2.15 - 2.14 (m, 3H), 2.09 - 2.02 (m, 2H), 1.92 (br s, 3H), 1.76 - 1.66 (m, 2H).

### Example 17. (1'R,2'R)-4,6-dihydroxy-N-(4-hydroxyphenethyl)-5'-methyl-2'-(prop-1-en-2-yl)-1',2',3',4'-tetrahydro-[1,1'-biphenyl]-3-carboxamide (compound 1q)]

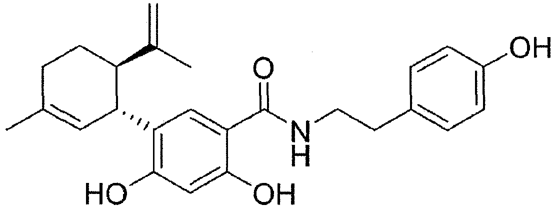

2,4-Dihydroxy-N-(4-hydroxyphenethyl)benzamide (1.23 mmol, 336.15 mg) was added to a 100 mL round flask and toluene: 1,4-dioxane = 6: 1 (35 mL) was added. After stirring until the mixture was dissolved, p-toluenesulfinic acid (0.5 mmol, 86.1 mg) was added at -10°C and (1S,4R)-1-methyl-4-(prop-1-en-2-yl)cyclohex-2-en-1-ol (1.36 mmol, 220.25 ul) was dissolved in toluene (2 mL), added dropwise, and stirred at room temperature for 2 hours. The reaction was terminated by adding an aqueous solution of sodium bicarbonate to the reaction mixture, and the mixture was extracted three times with diethyl ether. The extracted organic layer was dried using magnesium sulfate. The residue was purified through column chromatography to obtain compound **1q.** (Silica gel, Methanol/dichloromethane = 1:20, v/v) (white powder, 19 mg, 9% yield).

¹H NMR (400 MHz, MeOD-*d*₄) δ 7.40 (s, 1H), 7.07 (d, *J* = 8.52 Hz, 2H), 6.75 - 6.72 (m, 2H), 6.25 (s, 1H), 5.22 (s, 1H), 4.55 - 4.54 (m, 1H), 4.51 (s, 1H), 3.76 (d, *J* = 7.87 Hz, 1H), 3.52 (dt, *J* = 7.28, 2.56 Hz, 2H), 3.37 (s, 1H), 2.80 (t, J = 7.31 Hz, 2H), 2.43 - 2.37 (m, 1H), 2.23 - 2.17 (m, 1H), 2.02 (d, *J* = 17.28 Hz, 1H), 1.17 - 1.75 (m, 1H), 1.71 (br s, 3H), 1.66 (br s, 3H).

### Experimental example 1. Evaluation of acetylcholinesterase and butyrylcholinesterase inhibition efficiency of cannabidiol derivatives

Concentrations of 0, 2, 5, 10, 20, 50, 100, and 200 µM of the synthesized compounds were prepared by dilution in 10% DMSO, 63% phosphate buffer, and 27% tween 80, and 50 µl was dispensed into a 96-well plate. Acetylcholinesterase or butyrylcholinesterase was prepared at 0.5 U/ml, 50 µl was added to a 96-well plate, mixed with the aliquoted compound, and left for 10 minutes. The substrate, acetylthiocholine or butyrylthiocholine, was prepared at 0.4 mg/ml, then 50 µl was added to a 96-well plate and left for 20 minutes. Dithionitrobenzoic acid (DTNB) was prepared at 1.2 mg/ml, then 50 µl was added to a 96-well plate and left for 10 minutes. For an absorbance blank test, phosphate buffer was added instead of acetylthiocholine or butyrylthiocholine to correct for compound-induced absorbance values (positive control: rivastigmine).

### Experimental example 2. Evaluation of monoamine oxidase B (MAO-B) inhibition efficiency of cannabidiol derivatives

Samples were prepared by dilution with DMSO to give concentrations of 0, 2, 5, 10, 20, 50, 100, and 200 µM of the compound, and 5 µl was dispensed into a 96-well plate. 135 µl of potassium phosphate buffer (0.1 M, 7.4 pH) was dispensed into a 96-well plate. Monoamine oxidase B was prepared at 40 µl/ml, and 50 µl was added to the 96-well plate, mixed with the dispensed compound, and left for 20 minutes. The substrate, kynuramine solution, was prepared at 100 µM, then 10 µl was added to a 96-well plate and left for 10 min. The reaction was stopped by adding 75 µl of NaOH (2N). For the fluorescence blank experiment, potassium phosphate buffer was added instead of monoamine oxidase B to correct the compound-induced fluorescence intensity values.

The acetylcholinesterase (AChE), butyrylcholinesterase (BuChE), and monoamine oxidase B (MAOB) inhibition efficiency measured as above are summarized in Table 1 below with IC₅₀ values for each compound.

**[Table 1]**

| compound | Inhibition efficiency IC₅₀ (µM) | | |
|---|---|---|---|
| | IC₅₀ for AChE (µM) | IC₅₀ for BuChE (µM) | IC₅₀ for MAOB (µM) |
| 1a | 3.1 | 1.7 | 43.99 |
| 1b | 58.2 | 4.0 | 31.55 |
| 1c | 2.6 | 1.7 | 24.77 |
| 1d | 3.3 | 2.60 | N.D. |
| 1e | 20.0 | 9.3 | 26.21 |
| 1f | 20.0 | 19.3 | 29.88 |
| 1g | 74.0 | 8.6 | 61.11 |
| 1h | 78.0 | 55.3 | 35.77 |
| 1i | 6.6 | 7.3 | 16.44 |
| 1j | 2 | 30.6 | 141.56 |
| 1k | 5.3 | N.D. | N.D. |
| 1l | 23.3 | 6.6 | 7.99 |
| 1m | 42.6 | 44.6 | 117.67 |
| 1n | 10.6 | 13.3 | 30.67 |
| 1o | 15.3 | 27.3 | 56.1 |
| 1p | 4.6 | 2 | 2.1 |
| 1q | 7.3 | 3.3 | 18.1 |

| | | | |
|---|---|---|---|
| N.D.: Not Detected | | | |

As shown in Table 1 above, the synthesized cannabidiol derivatives were found to exhibit acetylcholinesterase and butyrylcholinesterase inhibitory activity as well as monoamine oxidase B inhibitory activity. In particular, a number of compounds exhibit butyrylcholinesterase inhibitory activity similar to that of the positive control rivastigmine [IC₅₀ values: acetylcholinesterase 1579.2 µM, butyrylcholinesterase 14.9 µ M], and a number of compounds exhibit acetylcholinesterase inhibitory activity superior to that of rivastigmine, suggesting that they may be useful in the treatment of cognitive dysfunction, including dementia.

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. For example, each of the components described in a single form may also be implemented in a distributed manner, and similarly, components described as distributed may also be implemented in a combined form.

The scope of the invention is indicated by the following patent claims. The meaning and scope of the patent claims and all modifications or variations derived from their equivalents are considered to be falling within the scope of the invention.

## Claims

1. A cannabidiol derivative compound represented by the following Formula 1, a hydrate thereof, a solvate thereof or a pharmaceutically acceptable salt thereof, wherein: 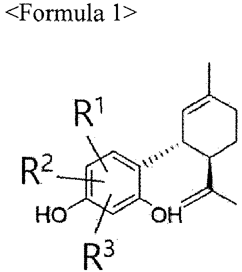
R¹, R², R³ are independently hydrogen, C₁-C₆ straight or branched chain alkyl, C₁-C₄ alkoxy, halogen, cyano, hydroxy, carboxyl, acetyl, dipentenyl, CONHR^{2a} or arylalkyl,
R^{2a} is arylalkyl substituted with hydroxy or alkoxy.

2. The cannabidiol derivative compound, a hydrate thereof, a solvate thereof or a pharmaceutically acceptable salt thereof of Claim 1, **characterized in that** the cannabidiol derivative compound represented by Formula 1 is represented by the following Formula 1-1: 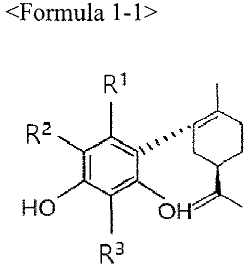 wherein R¹, R² or R³ is the same as defined in Claim 1.

3. The cannabidiol derivative compound, a hydrate thereof, a solvate thereof or a pharmaceutically acceptable salt thereof of Claim 1, **characterized in that** R¹ is hydrogen, methyl, propyl, pentyl, methoxy, bromo, cyano, hydroxy, acetyl or phenethyl.

4. The cannabidiol derivative compound, a hydrate thereof, a solvate thereof or a pharmaceutically acceptable salt thereof of Claim 1, **characterized in that** R² is hydrogen, chloro, CONHR^{2a} or dipentenyl.

5. The cannabidiol derivative compound, a hydrate thereof, a solvate thereof or a pharmaceutically acceptable salt thereof of Claim 1, **characterized in that** R^{2a} is methoxyphenethyl or hydroxyphenethyl.

6. The cannabidiol derivative compound, a hydrate thereof, a solvate thereof or a pharmaceutically acceptable salt thereof of Claim 1, **characterized in that** R³ is hydrogen, bromo, acetyl, carboxyl or dipentenyl.

7. The cannabidiol derivative compound, a hydrate thereof, a solvate thereof or a pharmaceutically acceptable salt thereof of Claim 1, **characterized in that** the cannabidiol derivative compound represented by Formula 1 is selected from the group consisting of:
[1] (1'R,2'R)-5'-methyl-6-pentyl-2'-(prop-1-en-2-yl)-1',2',3',4'-tetrahydro-[1,1'-biphenyl]-2,4-diol (1a),
[2] (1'R,2'R)-5'-methyl-2'-(prop-1-en-2-yl)-6-propyl-1',2',3',4'-tetrahydro-[1,1'-biphenyl]-2,4-diol (1b),
[3] (1'R,2'R)-5',6-dimethyl-2'-(prop-1-en-2-yl)-1',2',3',4'- tetrahydro-[1,1'-biphenyl]-2,4-diol (1c),
[4] (1'R,2'R)-5'-methyl-6-phenethyl-2'-(prop-1-en-2-yl)-1',2',3',4'-tetrahydro-[1,1-biphenyl]-2,4-diol (1d),
[5] (1'R,2'R)-6-bromo-5'-methyl-2'-(prop-1-en-2-yl)-1',2',3',4'- tetrahydro-[1,1'-biphenyl]-2,4-diol (1e),
[6] (1'R,2'R)-5-chloro-5'-methyl-2'-(prop-1-en-2-yl)-1',2',3',4'-tetrahydro-[1,1'-biphenyl]-2,4-diol (1f),
[7] (1'R,2'R)-4,6-dihydroxy-5'-methyl-2'-(prop-1-en-2-yl)-1',2',3',4'-tetrahydro-[1,1'-biphenyl]-2-carbonitrile (1g),
[8] (1'R,2'R)-6-methoxy-5'-methyl-2'-(prop-1-en-2-yl)-1',2',3',4'- tetrahydro-[1,1'-biphenyl]-2,4-diol (1h),
[9] 1-((1'R,2'R)-2,4-dihydroxy-5'-methyl-2'-(prop-1-en-2-yl)-1',2',3',4'-tetrahydro-[1,1'-biphenyl]-3-yl)ethan-1-one (1i),
[10] 1-((1R,1"R,2R,2"R)-2',4',6'-trihydroxy-5,5"-dimethyl-2,2"- di(prop-1-en-2-yl)-1,1",2,2",3,3",4,4"-octahydro-[1,1':3',1"- terphenyl]-5'-yl)ethan-1-one (1j),
[11] (1R,1"S,2R,2"S)-6'-methoxy-5,5"-dimethyl-2,2"-di(prop-1-en-2-yl)-1,1",2,2",3,3",4,4"-octahydro-[1,1':3',1"-terphenyl]-2',4'-diol (1k),
[12] 1-((1'R,2'R)-2,4,6-trihydroxy-5'-methyl-2'-(prop-1-en-2-yl)-1',2',3',4'-tetrahydro-[1,1'-biphenyl]-3-yl)ethan-1-one (11),
[13] (1'R,2'R)-3-bromo-5'-methyl-2'-(prop-1-en-2-yl)-1',2',3',4'-tetrahydro-[1,1'-biphenyl]-2,4-diol (1m),
[14] (1'*R*,2'*R*)-2,4-dihydroxy-5'-methyl-2'-(prop-1-en-2-yl)-1',2',3',4'-tetrahydro-[1,1'-biphenyl]-3-carboxylic acid (1n),
[15] (1'*R*,2'*R*)-4,6-dihydroxy-*N*-(4-methoxyphenethyl)-5'-methyl-2'-(prop-1-en-2-yl)-1',2',3',4'-tetrahydro-[1,1'-biphenyl]-3-carboxamide (1o),
[16] 1-((1'*R*,2'*R*)-4,6-dihydroxy-5'-methyl-2'-(prop-1-en-2-yl)-1',2',3',4'-tetrahydro-[1,1'-biphenyl]-2-yl)ethan-1-one (1p), and
[17] (1'*R*,2'*R*)-4,6-dihydroxy-*N*-(4-hydroxyphenethyl)-5'-methyl-2'-(prop-1-en-2-yl)-1',2',3',4'-tetrahydro-[1,1'-biphenyl]-3-carboxamide (1q).

8. A pharmaceutical composition for cognitive function improvement, comprising the cannabidiol derivative compound, a hydrate thereof, a solvate thereof or a pharmaceutically acceptable salt thereof of any one of Claims 1 to 7 as an active ingredient.

9. The pharmaceutical composition of Claim 8, **characterized in that** the cognitive function improvement is preventing or treating degenerative brain disease.

10. The pharmaceutical composition of Claim 9, **characterized in that** the degenerative brain disease is one or more selected from the group consisting of Alzheimer's disease, mild cognitive impairment, stroke and vascular dementia, frontotemporal dementia, Lewy body dementia, Creutzfeldt-Jakob disease, traumatic head injury, syphilis, acquired immunodeficiency syndrome and other viral infections, brain abscess, brain tumor, multiple sclerosis, dementia caused by metabolic diseases, hypoxia, Parkinson's disease, Lou Gehrig's disease, Huntington's disease, Pick's disease, amyotrophic lateral sclerosis, epilepsy, ischemia, stroke, attention deficit hyperactivity disorder, schizophrenia, depression, bipolar disorder, post-traumatic stress disorder, spinal cord injury, and myelitis.

11. A composition for inhibiting monoamine oxidase (MAO) comprising the cannabidiol derivative compound, a hydrate thereof, a solvate thereof or a pharmaceutically acceptable salt thereof of any one of Claims 1 to 7, as an active ingredient.

12. A food composition for cognitive function improvement comprising the cannabidiol derivative compound, a hydrate thereof, a solvate thereof or a pharmaceutically acceptable salt thereof of any one of Claims 1 to 7, as an active ingredient.

13. Method for preparing a cannabidiol derivative compound represented by the following Formula 1, comprising:
obtaining a first mixture by mixing a benzoic acid compound represented by Formula 2 below and cis-isolimonenol represented by Formula 3 below;
reacting the obtained first mixture with p-toluenesulfinic acid or oxalic acid hydrate to obtain a second mixture comprising a compound represented by Formula 4 below and a compound represented by Formula 1 below; and
purifying the obtained second mixture to obtain a cannabidiol derivative compound represented by Formula 1 below: 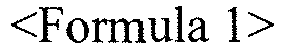 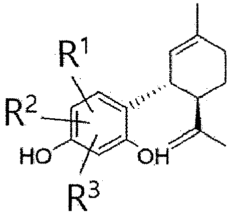 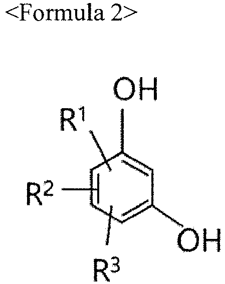 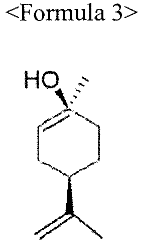 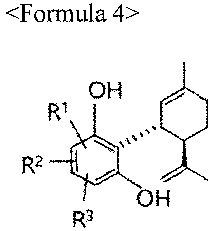
wherein in Formula 1, Formula 2, Formula 3 and Formula 4, R¹, R² and R³ are the same as defined in Claim 1.
